# EUROPEAN PATENT APPLICATION

(11) **EP 4 617 368 A1**
(43) Date of publication of application: **17.09.2025**
(21) Application number: 23869284.2
(22) Date of filing: 10.11.2023
(51) Int. Cl.: C12N 15/82, C12N 15/74, C12N 7/00

(54) **CUCUMBER MOSAIC VIRUS-BASED IMPROVED RECOMBINANT VECTOR**

(30) Priority: 11.11.2022 KR 20220150990; 23.03.2023 KR 20230038110
(71) Applicant: KT&G Corporation, Daedeok-gu Daejeon 34337 (KR); Seoul National University R&DB Foundation, Seoul 08826 (KR)
(72) Inventor: SEO, Hyo Seok, Daejeon 34128 (KR); SEO, Jang Kyun, Pyeongchang-gun, Gangwon-do 25354 (KR); LEE, Young Gi, Daejeon 34128 (KR); KWON, Sun Jung, Wonju-si, Gangwon-do 26479 (KR); KIM, Deuk Su, Daejeon 34128 (KR); LEE, Jeong Heon, Daejeon 34128 (KR); KIM, Myung Hwi, Pyeongchang-gun, Gangwon-do 25354 (KR)
(74) Representative: Ter Meer Steinmeister & Partner
(86) International application number: PCT/KR2023/018034
(87) International publication number: WO 2024/101936

(57) **Abstract**

Provided is a cucumber mosaic virus-based modified recombinant vector. The recombinant vector according to an example may significantly improve the efficiency of foreign protein expression in plants, and are more efficient at expressing a foreign protein than an existing recombinant vector, and thus may be widely utilized for the production of useful proteins in various plant species and for research thereon.

## Description

### Technical Field

This application claims priority to Korean Patent Application No. 10-2022-0150990, filed on November 11, 2022, and Korean Patent Application No. 10-2023-0038110, filed on March 23, 2023, the entire contents of which are hereby incorporated by reference.

The present disclosure relates to a cucumber mosaic virus (CMV)-based genetic recombination vector.

### Background Art

When a plant virus infects a host plant, the virus causes systemic infection within a few days and uses elements of the host to multiply its genome. During this proliferation process, the virus expresses its proteins in large quantities within plant cells. In addition, viruses have small genomes, therefore is possible to control a genome structure or gene expression mechanism through molecular biological manipulation.

Research is being actively conducted to develop gene delivery vectors by utilizing such characteristics of plant viruses to manipulate the genome structure of viruses to enable insertion and expression of foreign genes, but there is a continuous need to develop more efficient vectors.

Under this background, the inventers of the present disclosure have endeavored to develop a vector suitable for significantly increasing the expression level of a target protein, and have confirmed that a recombinant vector based on cucumber mosaic virus may significantly enhance the expression of a target protein, and have completed the present disclosure.

### Disclosure

### Technical Problem

One aspect is to provide a cucumber mosaic virus (CMV) recombinant vector including a nucleotide consisting of SEQ ID NO: 1.

Another aspect provides a transgenic microorganism transformed by the recombinant vector.

Another aspect is to provide a method of producing a protein, including a phase of preparing a cucumber mosaic virus recombinant vector including the nucleotide consisting of SEQ ID NO: 1; transforming the recombinant vector into a bacteria; and inoculating the transformed bacteria into a plant.

However, an objective to be solved in the present disclosure is not limited to the above-mentioned objective, and other objectives not mentioned can be clearly understood by those skilled in the art from the following description.

### Technical Solution

One aspect provides a cucumber mosaic virus (CMV) recombinant vector including a nucleotide consisting of SEQ ID NO: 1.

The cucumber mosaic virus recombinant vector may further include a nucleotide consisting of SEQ ID NO: 2, and may further include a nucleotide consisting of SEQ ID NO: 3. Although not limited thereto, the cucumber mosaic virus recombinant vector may include a nucleotide consisting of SEQ ID NO: 1, SEQ ID NO: 2, and SEQ ID NO: 3.

The cucumber mosaic virus (CMV) is a virus that occurs with the highest incidence in domestic pepper cultivation and causes serious damage, CMV is a positive-sense single stranded RNA virus with a wide host range that infects 885 plant species in 65 genera and consists of a three segmented genome. CMV isolates may be classified into subgroups IA, IB, and II according to nucleic acid sequence and among CMV isolates, a P0 type CMV-Fny (CMV-Fny strain) belongs to subgroup IA, and a P1 type CMV-GTN (CMV-GTN) strain) belongs to Subgroup IB. As used herein, CMV-GTN refers to a highly pathogenic P1 type, purely isolated from the pepper variety 'Cheongyang' grown by a pepper farmer in Gosan-gun, Chungcheongbuk-do in 2013 (Res. Plant Dis. 21(2): 99-102 (2015)).

According to an example, the nucleotide of the SEQ ID NO: 1 may include a multi-cloning site (MCS) operably linked to allow insertion of a foreign gene, and may further include a 2xFLAG tag and a 6xHIS tag after the MCS for detection and extraction of the expressed foreign protein. Accordingly, the protein produced by the recombinant vector may include the 2xFLAG tag and the 6xHIS tag at the C terminus.

As used herein, the term "recombinant vector" refers to a vector capable of expressing a targeted foreign gene in a host cell, including an essential regulatory element operably linked to cause expression of the gene insert. A suitable vector include an expression regulatory sequence such as a promoter, operator, initiation codon, termination codon, polyadenylation signal, and an enhancer, as well as a signal sequence or leader sequence for membrane targeting or secretion, and may be prepared in various ways depending on the purpose.

As used herein, the term "operably linked" refers to a gene requiring expression and a regulatory sequence thereof are linked together in a manner that enables gene expression.

Another aspect provides a transgenic microorganism, transformed by the recombinant vector.

In the transgenic microorganism, portions that overlap with the recombinant vector are omitted from description.

According to an example, the microorganism may be of a genus *Agrobacterium.*

Another aspect provides a method of producing a protein, including a phase of preparing the cucumber mosaic virus recombinant vector including the nucleotide consisting of SEQ ID NO: 1; transforming the recombinant vector into a bacteria; and inoculating the transformed bacteria into a plant.

The protein prepared by the above protein production method may include a 2xFLAG tag and a 6xHIS tag at the C terminus.

In the protein production method, overlapping portions of the recombinant vector and transgenic microorganism are omitted from the description.

### Advantageous Effects

With a cucumber mosaic virus-based recombinant vector according to an aspect, the efficiency of foreign protein expression in plants may be significantly increased. Since the recombinant vector according to an example has a significantly higher foreign protein expression efficiency than an existing recombinant vector, the recombinant vector may be widely used in the production and research of useful proteins in various plant species.

### Description of Drawings

FIG. 1A is a diagram illustrating a structure of pCMV-GTN-R1, pCMV-GTN-R2, and pCMV-GTN-R3 according to an example among the structures of a cucumber mosaic virus-based modified vector.
FIG. 1B is a diagram illustrating a structure of pCMV-R3V according to an example among the structures of the cucumber mosaic virus-based modified vector.
FIG. 1C is a diagram illustrating a structure of pCMV-R2V-B2 according to an example among the structures of cucumber mosaic virus-based modified vector.
FIG. 1D is a diagram illustrating a structure of pCMV-R3V-GFP bound with fluorescence to confirm the effect of pCMV-R3V according to an example among the structures of the cucumber mosaic virus-based modified vector.
FIG. 1E is a diagram illustrating a structure of pCMV-Fny-R1 and pCMV-Fny-R2 according to an example among the structures of the cucumber mosaic virus-based modified vector.
FIG. 1F is a diagram illustrating a structure of PZP-GFP according to an example among the structures of the cucumber mosaic virus-based modified vector.
FIG. 2A is a diagram illustrating a comparative experiment performed with PZP-GFP to evaluate the expression efficiency of the CMV-GTN-GFP combination vector (pCMV-GTN-R1 + pCMV-GTN-R2 + pCMV-R3V-GFP).
FIG. 2B is a diagram illustrating a comparative experiment performed with pCMV-Fny-R1 + pCMV-Fny-R2 + pCMV-R3V-GFP combination vector to evaluate the expression efficiency of the CMV-GTN-GFP combination vector (pCMV-GTN-R1 + pCMV-GTN-R2 + pCMV-R3V-GFP).
FIG. 3A is a diagram confirming the GFP expression level using a fluorescence measurement device to evaluate the expression efficiency of a CMV-GTN-B2-GFP combination vector (PCMV-R1 + PCMV-R2V-B2 + PCMV-R3V-GFP).
FIG. 3B is a diagram illustrating the results of SDS PAGE analysis by extracting an inoculation site protein on the second day after inoculation to evaluate the expression efficiency of the CMV-GTN-B2-GFP combination vector (pCMV-R1 + pCMV-R2V-B2 + pCMV-R3V-GFP).
FIG. 3C is a diagram illustrating quantitative analysis of a GFP band using ImageJ to evaluate the expression efficiency of the CMV-GTN-B2-GFP combination vector (pCMV-R1 + pCMV-R2V-B2 + pCMV-R3V-GFP).
FIG. 3D is a diagram illustrating the GFP expression level per fresh weight to evaluate the expression efficiency of the CMV-GTN-B2-GFP combination vector (pCMV-R1 + pCMV-R2V-B2 + pCMV-R3V-GFP).

### Best Mode

Hereinafter, the present invention will be described more specifically by Examples. However, the following Examples are provided only for illustrations and thus the present invention is not limited to or by them.

As used in the present specification, a singular form can include a plural form if it is not contextually indicated Also, the terms used in the present specification "comprise, include" and/or "comprising, including" specify shapes, numbers, steps, operations, members, elements described, and/or the existence of these groups, and do not exclude different shapes, numbers, operations, members, elements more than one, and/or the existence and addition of these groups.

### Example

### 1.Preparation of a cucumber mosaic virus-based modified vector

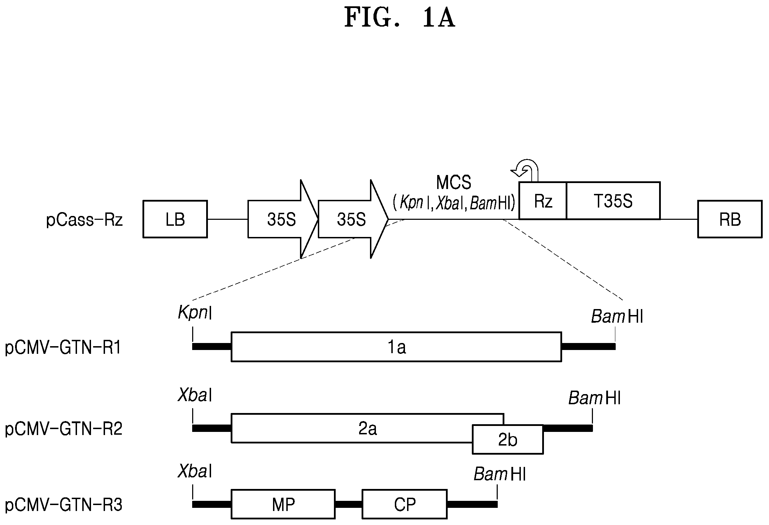

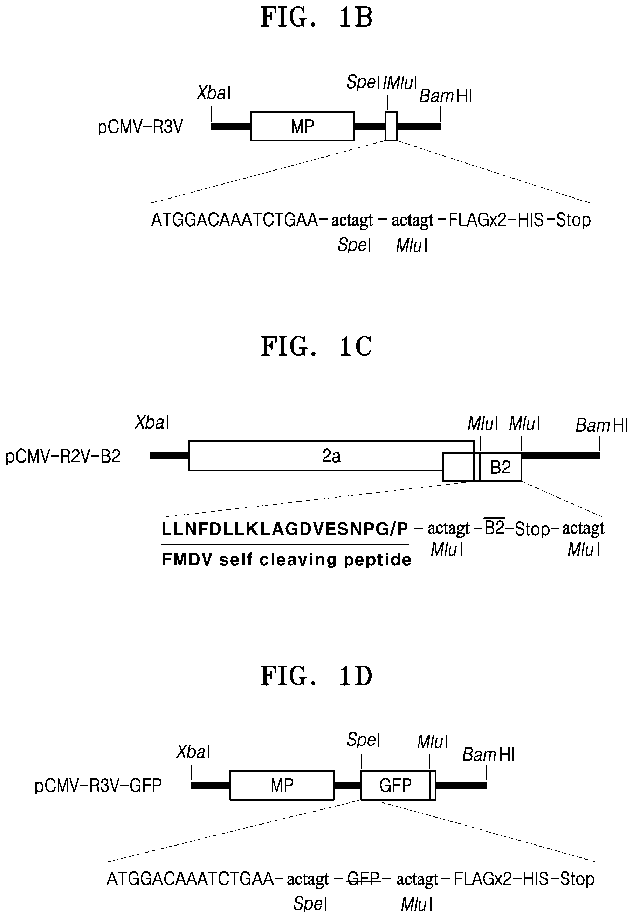

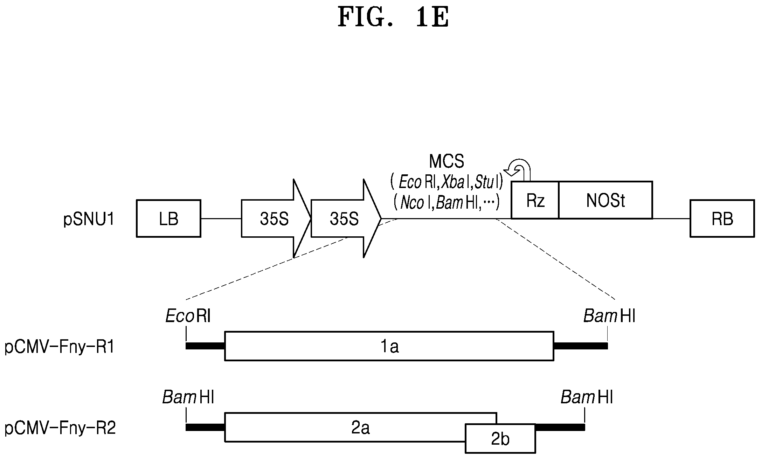

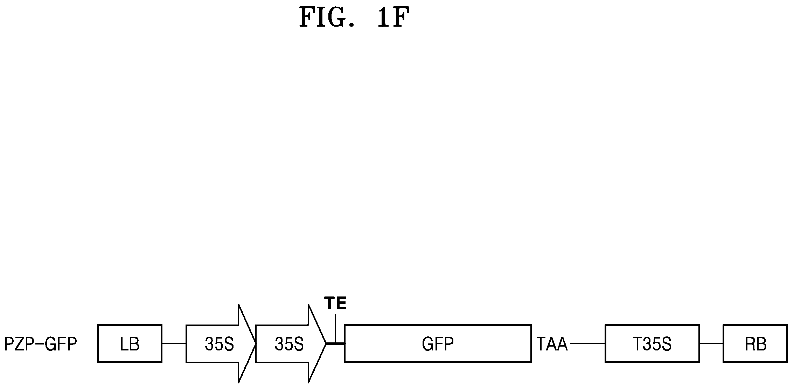

Hereinafter, a method for manufacturing a modified vector according to an example will be described with reference to FIG. 1. FIG. 1 schematically illustrates a method of preparing a cucumber mosaic virus-based modified vector according to an example. Specifically, a schematic diagram of a CMV infectious cDNA clone, a recombinant vector, and a recombinant clone constructed for GFP overexpression is shown.

A pCass-Rz vector was used as a binary vector to construct a cucumber mosaic virus (CMV)-based gene overexpression vector. The pCass-Rz vector includes, in the following order, a left border of T-DNA, double 35S promoter of CaMV, multiple cloning site (MCS; StuI, KpnI, XbaI, BamHI), cis-cleaving ribozyme sequence (Rz), 35S terminator (T), and right border of T-DNA, which may be transformed into *Agrobacterium* to deliver the target gene to plants through agroinfiltration. In addition, the pCass-Rz vector includes a kanamycin resistance gene, so transformants may be selectively cultured when transforming *E. coli* and *Agrobacterium.*

To construct a CMV-based recombinant protein overexpression vector, a previously isolated infectious cDNA clone (refer to Virus Evolution, Volume 6, Issue 2, July 2020, veaa070) of the CMV-GTN (Cucumber mosaic virus isolated from pepper; Res. Plant Dis. 21(2): 99-102(2015)) strain was used. The genome of CMV consists of RNA segments RNA1, RNA2, and RNA3. RNA1 encodes the 1a gene, which is involved in RNA replication, while RNA2 encodes the 2a gene, a replication enzyme, and the 2b gene, a gene silencing repressor. RNA3 encodes the viral movement protein (MP) and coat protein (CP) genes.

Genomic DNA for RNA1, RNA2, and RNA3 of the highly pathogenic CMV-GTN strain isolated from peppers grown domestically was amplified through RT-PCR, respectively. The PCR product for each genomic RNA were cloned using the restriction enzyme site present in the multi-cloning site (MCS) of the pCass-RZ vector. The resulting infectious cDNA clones for CMV-GTN RNA1, RNA2, and RNA3 were named pCMV-GTN-R1 (SEQ ID NO: 3), pCMV-GTN-R2 (SEQ ID NO: 4), and pCMV-GTN-R3 (SEQ ID NO: 5), respectively. In addition, pCMV-R3V refers to SEQ ID NO: 6, pCMV-R3V-GFP to SEQ ID NO: 7, pCMV-Fny-R1 to SEQ ID NO: 8, pCMV-Fny-R2 to SEQ ID NO: 9, and PZP-GFP to SEQ ID NO: 10.

Using an infectious clone of the CMV-GTN strain, a CMV-based vector was developed to overexpress foreign proteins by manipulating the genomic sequence of the CP open reading frame (ORF) of RNA3, which is expected to have high expression efficiency of foreign genes.

Specifically, the CP gene was removed from the RNA3 CP ORF and SpeI and MluI site were introduced as multi-cloning site (MCS) for insertion of foreign genes, and 2xFLAG tag and 6xHIS tag were introduced after the MCS for detection and extraction of the expressed foreign protein. Therefore, when cloning using MCS, the expressed protein includes a 2xFLAG tag and a 6xHIS tag at the C terminus. A CMV-GTN RNA3-based vector clone prepared in this way was named pCMV-R3V (SEQ ID NO: 1).

CMV RNA2 encodes the 2b gene, a gene silencing repressor, and in an example, a recombinant CMV RNA2 infectious clone was prepared in which the 2b gene was replaced with a B2 gene of flock house virus (FHV), which is known to be a more powerful gene silencing repressor , and was named pCMV-R2V-B2 (SEQ ID NO: 2).

Specifically, the C terminus of a 2b ORF of CMV RNA2 was removed and a foot and mouth disease virus (FMDV) self-cleaving peptide (LLNFDLLKLAGDVESNPG/P), a MluI site, and a FHV B2 sequence were introduced at the corresponding position. The B2 gene is expressed through translation of the 2b ORF, which is cleaved by the FMDV self-cleaving peptide and includes one additional proline (P) amino acid at the N-terminus.

### 2.Evaluation of expression efficiency of modified vector

### (1) Clone preparation to evaluate expression efficiency

To evaluate the efficiency of foreign gene expression in plants using the CMV-GTN-based vector prepared in the example 1. above, the preparation of a clone was intended by inserting the green fluorescent protein (GFP) gene using the SpeI and MluI restriction enzyme site of pCMV-R3V. For this purpose, a GFP gene was amplified through PCR using primers (5'-GGACTAGTATGTGGAGCAAGGGCGAGGAG-3' and 5'-AACGACCGTGTGAGGATCCCCTTGTACAGCTC-3'), treated with SpeI and MluI restriction enzymes, and inserted into the SpeI and MluI restriction enzyme site of the pCMV-R3V vector. The clone into which GFP was inserted was named pCMV-R3V-GFP (see FIG. 1). In addition, in order to prepare an infectious clone for the CMV-Fny strain, rather than the highly pathogenic CMV strain, the pCass-Rz vector was used to prepare pCMV-Fny-R1 and pCMV-Fny-R2 for CMV-Fny RNA1 and RNA2, respectively, using the same cloning method as pCMV-GTN-R1 and pCMV-GTN-R2. To use as a control group, PZP-GFP was prepared by inserting GFP into a PZP vector. The PZP vector include the left border of T-DNA, double 35S promoter of CaMV, Translation enhance element (TE), multiple cloning site (MCS; StuI, SpeI), 35S terminator (T), and right border of T-DNA in this order, which may transform *Agrobacterium* and transfer the target gene to plants through agroinfiltration. In addition, the PZP vector includes a spectinomy-cin resistance gene, so transformants may be selectively cultured when transforming *E. coli* and *Agrobacterium.* A PZP-GFP clone was prepared by inserting a GFP gene using a StuI and SpeI restriction enzyme site of the PZP vector.

The plasmid DNA of CMV-GTN-R1, pCMV-GTN-R2, pCMV-R3V-GFP, pCMV-R2V-B2, pCMV-Fny-R1, ppCMV-Fny-R2, and PZP-GFP clone with the structures shown in FIG. 1A to FIG. 1F were respectively transformed into *Agrobacterium* strain EHA105. The transformed *Agrobacterium* was shake-cultured in 5 ml of YEP liquid medium (including 100 µg/ml of kanamycin and 50 µg/ml of rifampicin) for 16 hours at 30 °C, 1 ml of the 1st culture solution was inoculated into 50 ml of fresh YEP medium (including 100 µg/ml of kanamycin, 50 µg/ml of rifampicin and 20 uM of acetosyringon) and shake-cultured for 6 hours at 30°C. The culture solution was centrifuged at 4800 G for 10 minutes to precipitate the *Agrobacterium* and suspended again in MMA infiltration buffer (MS salts, 10 mM MES, pH5.6, 200 uM acetosyringon) at a concentration of O.D. 0.7 at a wavelength of 600 nm. The suspension was then shake-cultured for 4 hours at 30°C.
- The *Agrobacterium* suspension transformed with each clone was mixed in equal proportions or alone and infiltrated under pressure using a 1 ml syringe on the adaxial surface of tobacco plant (*N. benthamiana*) leaf as follows.
   1) CMV-GTN-GFP: *Agrobacterium* suspension (O.D. 0.7) each transformed with pCMV-GTN-R1, pCMV-GTN-R2, and pCMV-R3V-GFP, were mixed in equal proportions and infiltrated.
   2) PZP-GFP: *Agrobacterium* suspension (O.D. 0.7) transformed with PZP-GFP was infiltrated alone.
   3) CMV-Fny-GFP: *Agrobacterium* suspension (O.D. 0.7) each transformed with pCMV-Fny-R1, pCMV-Fny-R2, and pCMV-R3V-GFP, were mixed in equal proportions and infiltrated.
   4) CMV-GTN-B2-GFP: *Agrobacterium* suspension (O.D. 0.7) each transformed with pCMV-GTN-R1, pCMV-R2V-B2, and pCMV-R3V-GFP, were mixed in equal proportions and infiltrated.

### (2) Evaluation of GFP expression efficiency

### 1) Expression efficiency of CMV-GTN-GFP combination vector

After inoculating each combination of *Agrobacterium* suspension, GFP expression over time at the inoculation site was observed using a fluorescence measurement device (FOBI system).

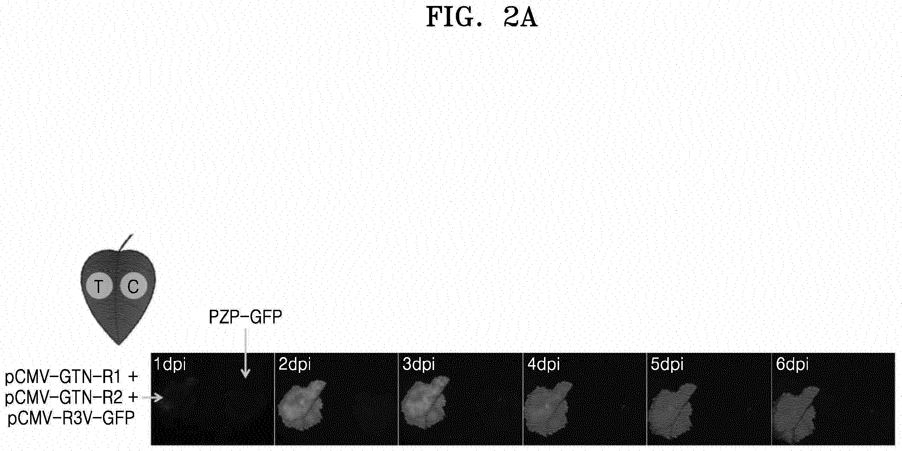

FIG. 2A is a diagram illustrating a comparative analysis of the GFP expression efficiency between the CMV-GTN-GFP combination (pCMV-GTN-R1 + pCMV-GTN-R2 + pCMV-R3VGFP) and PZP-GFP.

It was confirmed that compared to PZP-GFP, which was produced using a binary vector including the 35S promoter widely used in plant transformation, the CMV-GTN-GFP combination (pCMV-GTN-R1 + pCMV-GTN-R2 + pCMV-R3V-GFP) expressed a very large amount of GFP (see FIG. 2A). It was confirmed that the amount of GFP expressed by the CMV-GTN-GFP combination was highest at the second day after inoculation (2 dpi) and gradually decreased over time (see FIG. 2A).

Through the above results, it was confirmed that the vector including pCMV-R3V represented by SEQ ID NO: 1 may significantly enhance the expression of foreign protein in plants.

In addition, the CMV-GTN-GFP combination and the CMV-Fny-GFP combination (pCMV-Fny-R1 + pCMV-Fny-R2 + pCMV-R3V-GFP) were compared to determine whether the highly pathogenic CMV-GTN strain newly prepared in the example has a higher foreign protein expression efficiency as a vector compared to other CMV strains.

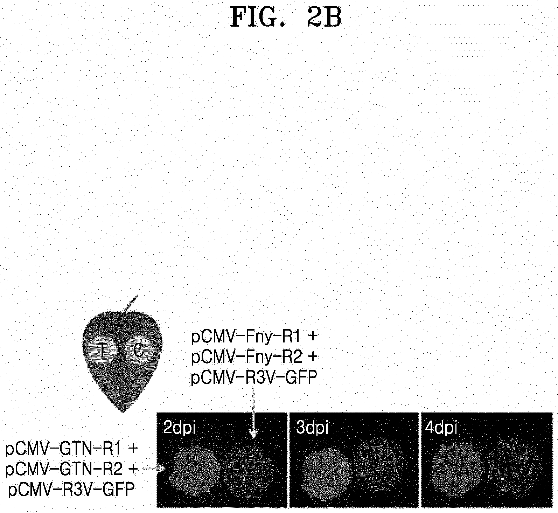

FIG. 2B shows a comparative analysis of GFP expression efficiency between the CMV-GTN-GFP combination and the CMV-Fny-GFP combination (pCMV-Fny-R1 + pCMV-Fny-R2 + pCMVR3V-GFP). After inoculating each combination of *Agrobacterium* suspension, GFP expression over time at the inoculation site was observed using a fluorescence measurement device (FOBI system).

As a result, it was confirmed that the CMV-GTN-GFP combination had a significantly superior GFP expression efficiency compared to the CMV-Fny-GFP combination (see FIG. 2B). These results show that the efficiency of foreign gene overexpression as a vector may vary depending on the CMV strain, and that the CMV-GTN strain used in the examples has excellent effectiveness as a foreign gene overexpression vector.

### 2) Expression efficiency of CMV-GTN-B2-GFP combination vector

Next, To determine whether using pCMV-R2V-B2, in which the CMV 2b gene was replaced with the FHV B2 gene, to express a more powerful gene silencing repressor increases expression efficiency, the CMV-GTN-GFP combination (pCMV-GTN-R1 + pCMV-R2V-B2 + pCMV-R3 V-GFP) was compared with the CMV-GTN-B2-GFP combination.

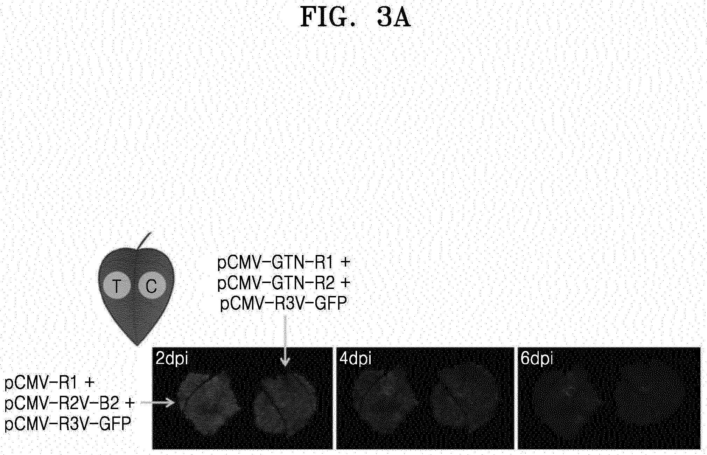

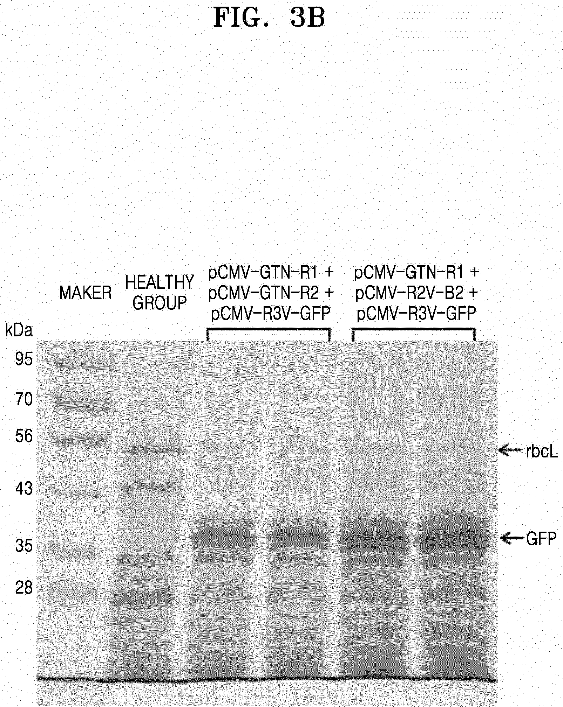

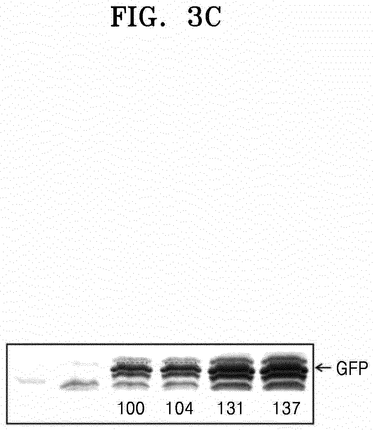

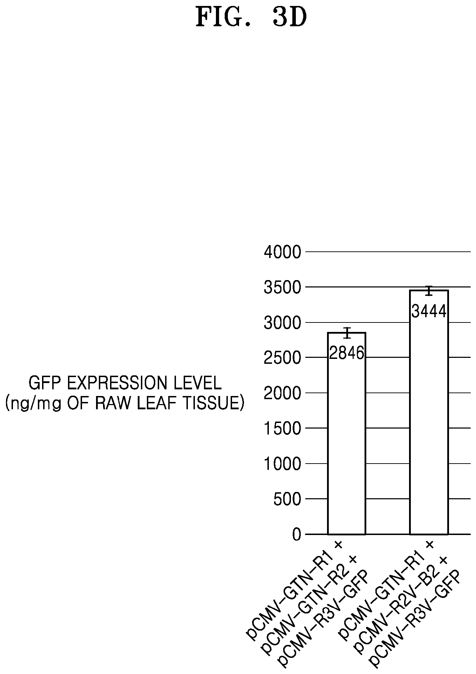

FIG. 3A shows GFP expression observed over time at the inoculation site after inoculation of each combination of *Agrobacterium* suspension using a fluorescence measurement device (FOBI system). FIG. 3B shows total protein extracted from the inoculation site at the second day after inoculation and analyzed by SDS PAGE. FIG. 3C is the result of quantitative comparative analysis of the GFP band of B using ImageJ. FIG. 3D shows the results of quantitative analysis of the GFP expression level of the recombinant CMV GTN-based vector compared to the fresh weight of the plant.

As a result of checking the expression efficiency in inoculated tobacco plants using a fluorescence measurement device (FOBI), it was confirmed that the CMV-GTN-B2-GFP combination had a somewhat higher expression efficiency compared to the CMV-GTN-GFP combination (see FIG. 3A). To more accurately compare the GFP expression efficiency between the two combinations, the total protein was extracted from the inoculation site on the second day after inoculation and subjected to SDS-PAGE analysis. As a result, it was confirmed that the amount of GFP protein expressed using the CMV-GTN-based vector accounted for a very high proportion of the total extracted proteins, additionally, it was confirmed that the CMV-GTN-B2-GFP combination induced somewhat more GFP expression compared to the CMV-GTN-GFP combination (FIG. 3B). As a result of analyzing such results with ImageJ, an image quantitative analysis program, it was confirmed that the CMV-GTN-B2-GFP combination had an increased GFP expression efficiency of about 30 % (see FIG. 3C).

To analyze the amount of GFP expression by the CMV-GTN-based vector compared to the plant fresh weight, cytoplasmic protein was extracted from the inoculation site on the second day after inoculation and quantitative analysis of GFP was performed using GFP Quantification Kit (abcam, UK, product number ab235672). In the case of the CMV-GTN-GFP combination, the GFP expression efficiency was 2846 ng per 1 mg of fresh weight, and in the case of the CMV-GTN-B2-GFP combination, the GFP expression efficiency was 3444 ng per 1 mg of fresh weight, which was increased by about 20%. (see FIG. 3D).

Therefore, the GFP gene was cloned into each vector and expressed in tobacco (*Nicotiana benthamiana*) plants, and the expression level was compared and analyzed, it was found that the highly pathogenic CMV-GTN-based vector showed a much higher level of GFP expression compared to the CMV-Fny strain generally used in research. In addition, it was confirmed that when pCMV-R2V-B2 was used compared to the wild-type CMV RNA2 infectious clone (pCMV-GTN-R2), the expression level of GFP was increased by about 12%.

From the above results, it may be seen that the CMV-GTN-B2-GFP combination (pCMV-GTN-R1 + pCMV-R2V-B2 + pCMV-R3V-GFP) vector according to an example show greatly improved expression efficiency in expressing foreign proteins in plants.

The descriptions of the above-described embodiments are merely examples, and it will be understood by one of ordinary skill in the art that various changes and equivalents thereof may be made. Therefore, the scope of the disclosure should be defined by the appended claims, and all differences within the scope equivalent to those described in the claims will be construed as being included in the scope of protection defined by the claims.

## Claims

1. A cucumber mosaic virus (CMV) recombinant vector comprising a nucleotide consisting of SEQ ID NO: 1.

2. The recombinant vector of claim 1,
further comprising a nucleotide consisting of SEQ ID NO: 2.

3. The recombinant vector of claim 1,
further comprising a nucleotide consisting of SEQ ID NO: 3.

4. The recombinant vector of claim 1,
wherein the cucumber mosaic virus is a P1 type cucumber mosaic virus.

5. The recombinant vector of claim 4,
wherein the P1 type cucumber mosaic virus is a CMV-GTN strain.

6. A transgenic microorganism, transformed by the recombinant vector of claim 1.

7. A method of preparing a protein, comprising: preparing a cucumber mosaic virus recombinant vector comprising a nucleotide consisting of SEQ ID NO: 1;
transforming the recombinant vector into bacteria; and
Inoculating the transformed bacteria into a plant.

8. The method of claim 7,
wherein the protein prepared by the method of preparing a protein comprises a 2xFLAG tag and a 6xHIS tag at the C terminus.
